Europäisches Patentamt

European Patent Office

Office européen des brevets

(11) Veröffentlichungsnummer: **0 488 024 A1**

## EUROPÄISCHE PATENTANMELDUNG

(21) Anmeldenummer: **91119720.0**

(51) Int. Cl.5: **A61K 49/04**

(22) Anmeldetag: **19.11.91**

(30) Priorität: **24.11.90 DE 9016028 U**

(43) Veröffentlichungstag der Anmeldung:
**03.06.92 Patentblatt 92/23**

(84) Benannte Vertragsstaaten:
**BE DE FR GB IT LU NL**

(71) Anmelder: **Klein, Hans-Martin, Dr. med.**
**Schurzelter Strasse 202**
**W-5100 Aachen(DE)**

(72) Erfinder: **Klein, Hans-Martin, Dr. med.**
**Schurzelter Strasse 202**
**W-5100 Aachen(DE)**

(74) Vertreter: **Bauer, Hubert, Dipl.-Ing.**
**Am Keilbusch 4**
**W-5100 Aachen(DE)**

(54) Hilfsmittel zur Untersuchung des Dünndarms.

(57) Als Hilfsmittel zur Untersuchung des Dünndarmes wird eine Kapsel (1) mit einer Füllung vorgeschlagen, die bei Kontakt mit $H_2O$ Gas freisetzt. Die Kapsel (1) ist mit einer säurefesten und magensaftresistenten Beschichtung (2) versehen. Die Beschichtung (2) gibt die Füllung (3) erst frei, nachdem die Kapsel (1) den Magen passiert hat und in den Dünndarm gelangt ist.

EP 0 488 024 A1

Die Erfindung betrifft ein oral einzuführendes Hilfsmittel zur Untersuchung des Dünndarmes.

Die radiologische Diagnostik des Magen-Darm-Traktes erfordert die Darstellung im Doppelkontrast. Dies ist für Speiseröhre, Magen und Dickdarm durch eine Gabe von bariumhaltigem Kontrastmittel und anschließender Luftinsufflation (Dickdarm) bzw. eine Gabe von $NaHCO_3$/Weinsäure-Pulver (Magen) möglich. Die Untersuchung des Dünndarmes erfordert derzeit das Einbringen einer Dünndarmsonde, über die zunächst Bariumbrei, danach Methylzellulose eingefüllt wird (Methode nach Sellink).

Die Plazierung der Sonde erfolgt unter Röntgenkontrolle und ist mit einer gewissen Strahlenbelastung verbunden. Die Einfüllung von Bariumbrei und Methylzellulose beinhaltet die Gefahr des Rückflusses in den Magen und die Speiseröhre mit dem Risiko des Übertrittes in die Atemwege (Aspiration).

Der Erfindung liegt die Aufgabe zugrunde, ein Hilfsmittel zur Untersuchung des Dünndarms vorzuschlagen, das die Plazierung einer Sonde und folglich auch die dabei erforderliche strahlenbelastende Röntgenkontrolle ebenso entbehrlich macht wie die Einfüllung von Bariumbrei und Methylcellulose.

Zur Lösung dieser Aufgabe wird ein Hilfsmittel vorgeschlagen, das erfindungsgemäß aus einer Kapsel mit einer Füllung besteht, die bei Kontakt mit $H_2O$ Gas freisetzt und eine Beschichtung aufweist, die so säurefest und magensaftresistent ist, daß die Füllung erst nach der Passage des Magens im Dünndarm das Gas freisetzt.

Durch das erfindungsgemäße Hilfsmittel wird erreicht, daß eine Doppelkontrastierung des Dünndarmes ohne vorheriges Legen einer Dünndarmsonde ermöglicht wird. Zudem entfällt die Gefahr eines Übertritts von Kontrastmittel in die Atemwege.

Nachfolgend sind zwei Ausführungsbeispiele beschrieben:

Ausführungsbeispiel 1:

I.) Gasbildendes Medium:
ZORU Granulat (Marushi Pharmaceutical Co. Ltd., Osaka/Japan)
3 g Granulat enthalten
1.05 g Weinsäure
1.05 g Natriumhydrogencarbonat
II.) Magensaftresistente Beschichtung:
EUDRAGIT L 12,5 P(Röhm Pharma GmbH, Weiterstadt/Deutschland)
III.) Beschichtungsverfahren:
Wirbelstrombeschichtung des Graunlates mit 15% Lack-Trockensubstanz.

Ausführungsbeispiel 2:

I.) Gasbildendes Medium:
UNIBARYT-Brausetabletten (Röhm Pharma GmbH, (Weiterstadt/Deutschland)
II.) Magensaftresistente Beschichtung:
EUDRAGIT L 12,5 P (Röhm Pharma GmbH, (Weiterstadt/Deutschland)
III.) Beschichtungsverfahren:
Wirbelstrombeschichtung der Brausetabletten mit 4 mg Lack-Trockensubstan/$cm^2$
In vitro Tests zur Magensaftresistenz nach DAB 9 zeigten folgende Resultate:

|  | Künstl. Magensaft | künstl. Darmsaft |
|---|---|---|
| Muster 1 (Granulat) | 2 Std. keine Reaktion | nach 5-10 min. Blasenbildung |
| Muster 2 (Tabletten) | 2 Std. keine Reaktion | nach 5-10 min. starke Blasenbildung |

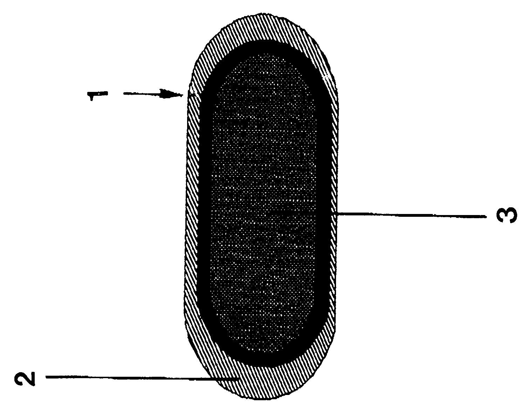

In der Zeichnung ist eine erfindungsgemäße Kapsel in einem Schnitt dargestellt:
Mit 1 ist die Kapsel insgesamt bezeichnet;
mit 2 die magensaftresistente (säurefeste) Beschichtung;
mit 3 die gasbildende Substanz, z. B. $NaHCO_3$/Tartrat.

**Patentansprüche**

1. Oral einzuführendes Hilfsmittel zur Untersuchung des Dünndarmes, gekennzeichnet durch eine Kapsel (1) mit einer Füllung (3), die bei Kontakt mit $H_2O$ Gas freisetzt, und einer Beschichtung (2), die so säurefest und magenresistent ist, daß die Füllung erst nach der Passage des Magens im Dünndarm das Gas freisetzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | CHEMICAL ABSTRACTS, vol. 92, 1980, Columbus, Ohio, US; abstract no. 153203, MIYAO,K ET AL: 'CONTRAST MEDIA FOR INTESTINAL RADIOGRAPHY' Seite 412 ; Spalte 1 ; * Zusammenfassung * | 1 | A61K49/04 |
| X | CHEMICAL ABSTRACTS, vol. 81, 1974, Columbus, Ohio, US; abstract no. 54466, NAOE,Y: 'FOAMING AGENTS FOR INTESTINAL X-RAY CONTRAST MEDIA' Seite 324 ; Spalte 1 ; * Zusammenfassung * | 1 | |
| A | US-A-3 131 123 (MASQUELIER) 28. April 1964 * das ganze Dokument * | 1 | |
| A | DATENBLATT(INFO L-2A),EUDRAGIT L12,5P ROEHM PHARMA GMBH,SEPTEMBER 1979 DARMSTADT,BRD. * das ganze Dokument * | 1 | RECHERCHIERTE SACHGEBIETE (Int. Cl.5 ) |
| | | | A61K |

Der vorliegende Recherchenbericht wurde für alle Patentansprüche erstellt

| Recherchenort | Abschlußdatum der Recherche | Prüfer |
|---|---|---|
| DEN HAAG | 26 FEBRUAR 1992 | SITCH W.D.C. |